# EUROPÄISCHE PATENTANMELDUNG

(11) **EP 1 634 603 A1**
(43) Veröffentlichungstag der Anmeldung: **15.03.2006**
(21) Anmeldenummer: 04020259.0
(22) Anmeldetag: 26.08.2004
(51) Int. Cl.: A61K 38/00, C12Q 1/48, C12Q 1/00

(54) **Behandlung von transformierten oder infizierten biologischen Zellen**

(71) Anmelder: Eberhard-Karls-Universität Tübingen Universitätsklinikum, 72076 Tübingen (DE)
(72) Erfinder: Stuhler, Gernot, Dr., 72070 Tübingen (DE); Salih, Helmut, Dr., 70199 Stuttgart (DE)
(74) Vertreter: Otten, Hajo

(57) **Zusammenfassung**

Die vorliegende Erfindung betrifft eine therapeutische oder/und diagnostische Substanz. Weiter betrifft sie einen Expressionsvektor, die Verwendung der Substanz oder/und des Expressionsvektors zur Herstellung einer pharmazeutischen Zusammensetzung, eine Zusammensetzung, ein Verfahren zur Diagnose einer Tumorerkrankung oder/und einer Infektion bei einem Lebewesen sowie ein Verfahren zur Behandlung einer Tumorerkrankung oder/und Infektion bei einem Lebewesen (Fig. 1).

## Beschreibung

Die vorliegende Erfindung betrifft eine therapeutische oder/und diagnostische Substanz. Weiter betrifft sie einen Expressionsvektor, die Verwendung der Substanz oder/und des Expressionsvektors zur Herstellung einer pharmazeutischen Zusammensetzung, eine Zusammensetzung, ein Verfahren zur Diagnose einer Tumorerkrankung oder/und einer Infektion bei einem Lebewesen sowie ein Verfahren zur Behandlung einer Tumorerkrankung oder/und Infektion bei einem Lebewesen.

Therapeutische und diagnostische Substanzen, die in der Therapie und Diagnose von Tumorerkrankungen oder Infektionen allgemein Verwendung finden, sind im Stand der Technik bekannt.

Ein therapeutischer Ansatz in der Behandlung von Tumor- und Infektionskrankheiten besteht in der Applikation von Medikamenten, die letztlich zu einer Schädigung bzw. einem Absterben oder Wachstumsstop der Tumorzellen bzw. infizierten Zellen führen sollen.

Bei den sogenannten Cytostatika handelt es sich um eine Gruppe von oft synthetisch hergestellten, chemisch heterogenen Substanzen, die auf unterschiedliche Art und Weise toxisch auf biologische Zellen wirken und Zellwachstum und -teilung hemmen.

Die bisher verfügbaren cytostatischen bzw. cytotoxischen Substanzen wirken allerdings nicht selektiv auf die Tumorzellen, sondern schädigen auch gesundes Gewebe. Davon sind besonders Gewebe mit hoher Zellteilungsrate, wie z. B. Keimdrüsen, Haarfollikel und Zellen des blutbildenden Systems, betroffen. Ein Überblick über die Entwicklung von Cytostatika findet sich in S. N. Gardner und M. Fernandes (2004), Cytostatic Anticancer Drug Development, J. Exp. Ther. Oncol., Seiten 9 bis 18.

Ein Fortschritt in der Behandlung und Diagnose von Tumor- und Infektionskrankheiten konnte aufgrund der Entdeckung von Antigenen erzielt werden, die auf der Oberfläche von infizierten bzw. transformierten Zellen exprimiert werden. Man bezeichnet derartige Oberflächenproteine auf Tumorzellen auch als sogenannte Tumorantigene. Basierend auf diesen Erkenntnissen gibt es Bemühungen, Substanzen zu entwickeln, die gezielt derartige Tumorantigene erkennen und darüber einen selektiven Angriff auf die Tumorzelle vermitteln. Dies wird bspw. über für die Tumorantigene spezifische Antikörper versucht, die mit cytotoxischen Substanzen gekoppelt sind, oder auch über die gezielte Stimulierung des Immunsystems gegen Tumorzellen durch Applikation von ggf. modifizierten Tumorantigenen bzw. der direkten Verabreichung von sogenannten Tumorvakzinen. Ein Überblick zu diesem therapeutischen Ansatz ist in Joseph N. Blattmann und Philip D. Greenberg (2004), Cancer Immunotherapy: A Treatment for the Masses, Science, Vol. 305, Seiten 200 bis 205, dargestellt.

Nachteilig bei diesem Ansatz ist jedoch, dass die meisten der zurzeit bekannten Tumorantigene keine klare Abgrenzung gegenüber benignen Erkrankungen oder auch gegenüber gesunden Zellen erlauben und damit ein gezielter Angriff bspw. von bösartigen Tumoren oftmals nicht oder nur unzureichend möglich ist. Des Weiteren sind infizierte oder transformierte Zellen beschrieben worden, die keine besondere Immunogenität zeigen. Eine Unterscheidung dieser Zellen gegenüber gesunden Zellen über Oberflächenmarker und ein darüber vermittelter gezielter therapeutischer Eingriff ist dann nicht möglich.

Im Stand der Technik ist auch bekannt, dass in Tumorzellen gegenüber normalen Zellen regulatorische Mechanismen verändert sind, die ihre Ursache in genetischen Alterationen verschiedener Signaltransduktionsfaktoren haben können. Eine zusammenfassende Übersicht über genetische Veränderungen in Tumorzellen findet sich in Douglas Hanahan und Robert A. Weinberg (2000), The Hallmarks of Cancer, Cell, Vol. 100, Seiten 57 bis 70.

In der Fachwelt ist bekannt, dass in bestimmten Tumorzellen permanente oder verstärkte Wachstumssignale von strukturell zwar intakten aber amplifizierten Oberflächen-Rezeptorkinasen in die Zelle geleitet werden, wohingegen in gesunden Zellen nur zu bestimmten Zeiten im Zellzyklus Wachstumsimpulse induziert werden. Gleichermaßen sind bei einer Vielzahl von Tumoren aktivierende Mutationen an einzelnen intrazellulären Faktoren der Signaltransduktionskette beschrieben worden, wie bspw. an dem Ras-Protein, einer monomeren GTPase mit proliferationsregulierender Aktivität. Das Ras-Protein liegt in 30 % der menschlichen Tumoren mutiert vor. Diese vor allem im exokrinen Pankreaskarzinom und im Colonkarzinom beschriebene Mutation führt zum Verlust der hydrolysierenden Aktivität von Ras und ruft eine permanent aktive und damit proliferationsstimulierende Form des Ras-Proteins hervor. Aber auch die Inhibition oder Ausschaltung von wachstumshemmenden Faktoren, wie dem Retinoblastoma- (Rb) oder dem p53-Protein, den sogenannten Tumorsuppressoren, wird in Tumorzellen beobachtet. Beschrieben wird auch eine Veränderung der Telomerstruktur in Tumorzellen, die im Zusammenhang mit dem Erwerb von deren immortalisierenden Eigenschaften steht. Diese Zellen zeichnen sich dadurch aus, dass sie, im Gegensatz zu normalen Zellen, dauerhaft in Kultur genommen werden können. Weitere zusammenfassende Darstellungen hierzu finden sich in William C. Hahn und Robert A. Weinberg (2002), Rules for Making Human Tumor Cells, N. Engl. J. Med., Vol. 347, No. 20, Seiten 1593 bis 1603; oder in William C. Hahn und Robert A. Weinberg (2002), Modelling the Molecular Circuitry of Cancer, Nat. Rev. Cancer , Vol. 2(5), Seiten 331 bis 341.

Irish et al. (2004), Single Cell Profiling of Potentiated Phospho-Protein Networks in Cancer Cells, Cell, Vol. 118, Seiten 217 bis 228, beschreiben, dass Signaltransduktionsmechanismen, die über die Phosphorylierung von Signalmolekülen ablaufen, in Tumorzellen allgemein verändert sind. Die Autoren erstellen aufgrund dieser Beobachtung tumorspezifische multidimensionale molekulare Phosphoprofile, beschreiben jedoch nicht, in welcher Form Signaltransduktionsfaktoren in Tumorzellen gegenüber Nicht-Tumorzellen verändert sind. Erst recht wird nicht beschrieben, in welcher Beziehung die Signalmoleküle zum Zellzyklus stehen, da die dort beschriebenen Experimente nur über einen sehr kurzen Zeitraum durchgeführt wurden.

Trotz dieser Erkenntnisse über die in Tumorzellen veränderten Signaltransduktionsmechanismen ist es bislang nicht gelungen, eine Substanz bereitzustellen, die diese Veränderungen therapeutisch oder/und diagnostisch nutzt.

Aufgabe der vorliegenden Erfindung ist es deshalb, eine Substanz bereitzustellen, mit der zielgerichtet transformierte oder/und infizierte biologische Zellen erkannt oder/und bekämpft werden können und mit der die Nachteile der bekannten Substanzen weitgehend vermieden werden.

Diese Aufgabe wird durch die Bereitstellung einer therapeutischen oder/und diagnostischen Substanz gelöst, die mittelbar oder unmittelbar mit zumindest zwei Molekülen, die ausschließlich in einer transformierten oder/und infizierten biologischen Zelle im Wesentlichen zeitgleich auftreten, derart reagiert, dass sie eine biologische oder/und detektierbare Eigenschaft induziert.

Erfindungsgemäß wird unter einer Substanz sowohl eine rein chemisch definierte Substanz, bspw. eine organische oder anorganische Verbindung, als auch eine biologische Substanz, bspw. ein Peptid oder Protein, verstanden. Es kann sich deshalb auch um einen niedermolekularen Wirkstoff, ein sogenanntes "Small molecule" oder um ein virales ggf. molekular modifiziertes Partikel oder um einen Antikörper handeln.

Unter therapeutischer bzw. diagnostischer Substanz ist eine solche zu verstehen, die zur Anwendung in der Heilbehandlung bzw. Diagnose vorgesehen ist.

Unter einer transformierten Zelle wird erfindungsgemäß eine solche verstanden, bei der eine maligne, neoplastische bzw. onkogene Transformation, d.h. eine solche Veränderung der Zellen stattgefunden hat, die zu einem veränderten Wachstumsverhalten führt. Ursachen hierfür können chemische oder physikalische Noxen sowie onkogene Viren sein. Auch werden spontane Mutationen, die zur Transformation einer Zelle führt, beobachtet. Transformierte Zellen erlangen häufig die Fähigkeit zur Bildung von Tumoren.

Unter infizierter Zelle wird erfindungsgemäß eine solche verstanden, in die Krankheitserreger, wie bspw. Viren, Bakterien, Pilze bzw. Mikroorganismen aller Art oder Teile hiervon, eingedrungen sind oder/und zu einer Veränderung der Zelle geführt haben. Insbesondere wird in diesem Zusammenhang an eine Infektion der Zellen durch onkogene Viren gedacht, wie z.B. an sogenannte DNA-Tumorviren: bestimmte Adenoviren, Papillomaviren, Herpesviren, wie das Epstein-Barr-Virus (EBV). Für EBV ist bspw. gezeigt worden, dass es nach einer Infektion zur Expression von Kinasenhomologen in der Zelle kommt, die in die Regulation der Signaltransduktion eingreifen. Gedacht wird bei infizierenden Krankheitserregern auch an Vertreter der sogenannten RNA-Tumorviren oder Retroviren sowie auch an solche Organismen allgemein, die in die Signaltransduktionsmechanismen der infizierten Zelle eingreifen und diese verändern.

Erfindungsgemäß ist aber auch eine solche Zelle umfasst, die in unmittelbarem oder mittelbarem Kontakt zu transformierten bzw. infizierten Zellen steht, selbst jedoch nicht zwingend transformiert bzw. infiziert sein muss. Eine derartige Zelle steht jedoch unter dem Einfluss der veränderten Zelle und kann zum Fortschreiten einer Tumor- oder infektiösen Krankheit ganz entscheidend beitragen. Diese Zelle ist deshalb bspw. als therapeutisches "Target" besonders interessant.

Unter den zumindest zwei Molekülen, mit denen die erfindungsgemäße Substanz reagiert, werden zelleigene Verbindungen verstanden, wie z.B. Enzyme, die sich in Ihrer Aktivität oder/und Spezifität oder/und Affinität oder/und Zugänglichkeit zu bzw. für Reaktionspartner oder sonstigen Eigenschaften voneinander unterscheiden. Diese Moleküle treten nach Erkenntnissen der Erfinder in gesunden Zellen, d.h. in nicht-transformierten oder nicht-infizierten Zellen nicht gleichzeitig auf. Diese in einzelnen gesunden Zellen gegenüber einzelnen transformierten bzw. infizierten Zellen zu beobachtenden Unterschiede im zeitlichen Auftreten der zwei Moleküle können bspw. auf zellzyklusspezifische Regulationsmechanismen zurückgehen. So ist bekannt, dass bspw. cyclinabhängige Kinasen (engl. cyclin-dependent kinases, CDK) über den Zellzyklus hinweg sowohl in ihrer Aktivität als auch in ihrer Verfügbarkeit reguliert werden, so dass diese nur zu bestimmten Zeiten im Zellzyklus auftreten. Das Phänomen des nicht gleichzeitigen Auftretens der in Rede stehenden Moleküle in einzelnen gesunden Zellen kann aber auch auf sonstige regulatorische intra- und extrazelluläre Phänomene, wie z.B. zeitlich koordinierte mitogene Impulse, zurückgehen.

Gleichzeitiges Auftreten der beiden Moleküle in einer transformierten oder/und infizierten Zelle bedeutet erfindungsgemäß, dass diese zwei Moleküle in einer gesunden Zelle entweder nicht zeitgleich vorhanden sind, nicht zeitgleich aktiv sind bzw. ihre Aktivität nicht zeitgleich oder in gleicher Art und Weise entfalten können. D.h. gleichzeitig in diesem Sinne bedeutet, dass diese zwei Moleküle in einer transformierten bzw. infizierten Zelle über längere Zeiten hinweg im Zellzyklus, nicht etwa nur punktuell, oder auch im arretierten Zustand, d.h. in der G0-Phase, im Wesentlichen zeitgleich vorhanden oder zeitgleich aktiv sind.

Diese Gleichzeitigkeit in transformierten oder/und infizierten Zellen bedeutet erfindungsgemäß, dass die zwei Moleküle in jeder einzelnen dieser Zellen im Wesentlichen zeitgleich auftreten.

Die Reaktion der Substanz mit den zumindest zwei Molekülen kann direkt erfolgen, d.h. durch unmittelbare sterische Interaktion der Substanz mit den zwei Molekülen, aber auch indirekt bspw. über zwischengeschaltete Faktoren bzw. zwischengeschaltete Moleküle.

Die Reaktion der erfindungsgemäßen Substanz mit den zwei Molekülen kann bspw. zu einer Anlagerung oder Abspaltung von Molekülen oder Molekülgruppen, wie Phosphatgruppen, an die bzw. von der Substanz oder an bzw. von zwischengeschalteten Faktoren führen, zu einer Umlagerung von Gruppen oder Abschnitten an der Substanz oder an zwischengeschalteten Faktoren.

Unter biologischer Eigenschaft ist eine solche zu verstehen, die spezifisch aufgrund der Reaktion mit den zumindest zwei Molekülen durch die Substanz induziert wird und sich bspw. als biologische Aktivität in der Zelle darstellt. Allgemein kann es sich um eine enzymatische aber auch um eine sonstige chemische, biochemische oder physikalische Aktivität handeln, die durch die Substanz induziert wird.

Unter detektierbarer Eigenschaft ist erfindungsgemäß eine solche zu verstehen, die durch die Reaktion der Substanz mit den zumindest zwei Molekülen induziert wird und sich als messbare Größe darstellt, die mittelbar oder unmittelbar von der so reagierten Substanz ausgeht. Als detektierbare Eigenschaft kommt eine jegliche Eigenschaft in Frage, z.B. eine Aktivität, die mittels im Stand der Technik bekannter chemischer, biochemischer oder physikalischer Analysemethoden erfasst werden kann.

Die biologische oder/und detektierbare Eigenschaft kann dadurch induziert werden, dass die Substanz nach der Reaktion mit den zumindest zwei Molekülen in der Zelle verstoffwechselt wird, in einen Zustand einer anderen Aktivität überführt wird, eine andere Struktur einnimmt, die Expression eines zelleigenen Produktes wie bspw. eines Enzymes bewirkt oder die Aktivität eines zelleigenen Proteins modifiziert.

Das Ergebnis der Induktion der biologischen oder/und detektierbaren Eigenschaft kann sich dann darin darstellen, dass das Stoffwechsel- oder exprimierte Produkt oder auch die reagierte erfindungsgemäße Substanz entweder selbst therapeutisch bzw. diagnostisch wirkt, oder aber indirekt eine Reaktion auslöst, wie bspw. eine Immunreaktion, oder als Mediator den zielgerichteten Angriff eines Therapeutikums oder Diagnostikums ermöglicht.

Erfindungsgemäß wird die vorstehend näher erläuterte biologische und/oder detektierbare Eigenschaft aufgrund einer ins Belieben des Fachmanns gestellten geeigneten Konstruktion der Substanz nur dann induziert, wenn diese im Wesentlichen zeitgleich mit den zumindest zwei Molekülen reagiert. Eine Reaktion der erfindungsgemäßen Substanz mit nur einem der zwei Moleküle führt nicht zur Induktion der biologischen oder/und detektierbaren Eigenschaft.

Die der Erfindung zugrunde liegende Aufgabe wird damit vollkommen gelöst.

Die Erfinder haben nämlich erkannt und erstmals auf Einzelzellebene nachweisen können, dass in einer transformierten und auch infizierten biologischen Zelle intrazelluläre Moleküle, wie bspw. Faktoren der Signaltransduktionskaskade, im Wesentlichen zeitgleich auftreten, bspw. im Zellzyklus über eine längere Zeit hinweg gleichzeitig aktiv sind, wohingegen diese Moleküle in einer gesunden Zelle in einer zeitlich deutlich unterscheidbaren Abfolge auftreten, bspw. im Zellzyklus zu unterschiedlichen Zeiten aktiv sind.

Dieses von den Erfindern exklusiv bei transformierten bzw. infizierten Zellen erkannte Phänomen der Gleichzeitigkeit des Auftretens von Molekülen ist im Stand der Technik nicht beschrieben. So ist zwar bekannt, dass in größeren Populationen von Tumorzellen, wie bspw. Zellkulturen, Gewebeverbänden oder ganzen Organen, bestimmte Signalmoleküle konstitutiv aktiv sind, bspw. die Ras-Kinase in 30 % aller Tumoren dauerhaft Signale in die Zelle sendet. Bei Betrachtung einer Vielzahl von transformierten Zellen kann deshalb ein paralleles Auftreten der Ras-Kinase und anderen Signalmolekülen, wie CDKs, wenn auch nur punktuell im Zellzyklus, angenommen werden. Diese Annahme im Stand der Technik ließ jedoch keinerlei Rückschlüsse auf Verhältnisse in einer einzelnen Zelle zu und verhinderte damit bislang die Konzipierung einer zielgerichteten Substanz, die in jeder einzelnen transformierten oder/und infizierten Zelle wirksam ist.

Auf der Grundlage der neuen an einzelnen Zellen gewonnenen Erkenntnisse, war es den Erfindern nun erstmals möglich, die erfindungsgemäße Substanz zu konzipieren, mit der gezielt in einzelnen transformierten und infizierten Zellen aufgrund einer im Wesentlichen zeitgleichen Reaktion mit den zwei Molekülen eine biologische oder/und diagnostische Eigenschaft induziert werden kann.

Diese Eigenschaft wird in gesunden Zellen nicht induziert, da wegen des dort zeitlich deutlich unterschiedlichen Auftretens der zumindest zwei Moleküle keine entsprechende Reaktion stattfindet. In gesunden Zellen kommt es stattdessen ggf. lediglich zu einer Reaktion der erfindungsgemäßen Substanz mit nur einem Molekül, da das jeweils andere Molekül dann nicht aktiv, vorhanden oder zugänglich ist, bspw. aufgrund zellzyklusspezifischer Regulationen.

Die Substanz kann so ausgestaltet sein, dass eine Reaktion der Substanz mit nur einem Molekül, eine hinreichend zeitlich versetzte Reaktion zuerst mit dem einen und dann mit dem anderen Molekül, oder eine gänzlich fehlenden Reaktion mit den zwei Molekülen zur Instabilität, zum direkten oder indirekten Abbau, zur Inaktivierung, der Ausschleusung oder sonstigen Funktionsunfähigkeit der erfindungsgemäßen Substanz führt und somit die Induktion der biologischen oder/detektierbaren Eigenschaft in gesunden Zellen gänzlich ausbleibt.

Die Erfinder stellen somit eine Substanz bereit, die hochselektiv und spezifisch in transformierten bzw. infizierten Zellen Effekte induziert, diese Effekte jedoch in gesunden Zellen im Wesentlichen nicht induziert. Die Substanz stellt deshalb ein wertvolles Tool in der Therapie und Diagnostik von Tumor- und infektiösen Krankheiten dar.

Die erfindungsgemäße Substanz ist vorzugsweise so ausgestaltet, dass diese mit zwei zellulären Enzymen reagiert, insbesondere mit Kinasen, die in die Regulation des Zellzyklus involviert sind.

Die vorstehende Maßnahme hat den Vorteil, dass Schlüsselfaktoren der Regulation des Zellzyklus ausgenutzt werden, um die biologische oder/und detektierbare Eigenschaft der Substanz zu induzieren. Wie die Erfinder nämlich herausgefunden haben, sind bspw. zwei Enzyme, vorzugsweise zwei Kinasen bzw. Phosphotransferasen in einer transformierten oder infizierten Zelle im Wesentlichen zeitgleich aktiv, nicht jedoch in einer gesunden Zelle.

Die ins Belieben des Fachmanns gestellte Ausgestaltung der erfindungsgemäßen Substanz, so dass diese nach Reaktion mit den zwei Enzymen bzw. zwei Kinasen in einer Zelle die biologische oder/und detektierbare Eigenschaft induziert, ist deshalb besonders zweckmäßig, weil dadurch ein für eine transformierte oder infizierte Zelle hochselektives therapeutisches oder diagnostisches Tool bereitgestellt wird. Auch kann mit dieser Maßnahme eine biologische Eigenschaft in einer solchen Zelle induziert werden, die mit in Stand der Technik beschriebenen Viren infiziert wurde, die eine Aktivierung von zellzyklusregulierenden Kinasen bewirkt. Erfindungsgemäß sind aber auch solche Kinasen erfasst, die in zellzyklusarretierten Zellen auftreten, d.h. in solchen Zellen, die sich in der G0-Phase befinden.

Nach einer bevorzugten Weiterentwicklung ist die erfindungsgemäße Substanz derart ausgestaltet, dass die biologische oder/und detektierbare Eigenschaft dann induziert wird, wenn eines der zwei Moleküle ein Enzym der Ras/Raf-Signaltransduktionskette darstellt, und das andere der zwei Moleküle ein Enzym der CDK2-Signaltransduktionskette darstellt.

Der Ras/Raf-Signaltransduktionsweg führt über eine Kaskade von im Wesentlichen Phosphorylierungsereignissen zu einer Aktivierung der MAP-Kinase (mitogenaktivierte Proteinkinase, auch ERK1 genannt; vgl. William C. Hahn und Robert A. Weinberg (2002), N. Engl. J. Med. (a.a.O.). Der CDK2-Signaltransduktionsweg führt über die Stimulation der Transkription zu einer Aktivierung der cyclinabhängigen Kinase 2. Erfindungsgemäß kann die CDK2-Kinase sowohl aus der katalytischen Untereinheit cdk2 und der regulatorischen Untereinheit Cyclin A als auch aus cdk2 und Cyclin E bestehen. Ferner kommt erfindungsgemäß eine jegliche aktive CDK2-Kinase in Frage; vgl. hierzu A. W. Murray (2004), Recycling the Cell Cycle: Cyclins Revisited, Cell, Vol. 116 (2), Seiten 221 bis 234.

Diese bevorzugte vorstehende Maßnahme ist deshalb vorteilhaft, da hiermit ein von den Erfindern erstmals erkanntes exklusiv in transformierten und infizierten Zellen auftretendes Phänomen therapeutisch und diagnostisch ausgenutzt wird. So wird im Stand der Technik angenommen, dass zwar die Ras-Kinase in Kulturen aus Tumorzellen oder in sonstigen Tumorzellpopulationen insgesamt betrachtet konstitutiv aktiv ist, die Kinetik der Kinaseaktivitäten auf Einzelzellebene jedoch bislang völlig unklar ist. Nun wurde aber zum ersten mal erkannt und auf Einzelzellebene nachgewiesen, dass in transformierten und infizierten Zellen sowohl der Ras/Raf- als auch der CDK2-Signaltransduktionsweg im Wesentlichen zeitgleich über längere Zeiten abläuft, was zum zeitgleichen Vorhandensein der Aktivitäten der einzelnen Faktoren der jeweiligen Signalkette, wie bspw. der MAP-Kinase und der CDK2-Kinase, in der transformierten bzw. infizierten Zelle führt.

Diese Erkenntnis war deshalb besonders überraschend, da in gesunden Zellen beide Signaltransduktionswege zeitlich deutlich versetzt ablaufen. So ist die CDK2-Kinase im Komplex mit Cyclin E in gesunden Zellen üblicherweise in der späten G1-Phase bzw. zu Beginn der S-Phase, in der die DNA-Replikation stattfindet, und im Komplex mit Cyclin A etwas später in der S-Phase aktiv.

Die MAP-Kinase hingegen ist in der gesunden Zellen i.d.R. in der sehr frühen G1-Phase, nicht mehr jedoch zu Beginn oder während der S-Phase aktiv. Daraus resultiert, wie die Erfinder zeigen erstmals konnten, in der gesunden menschlichen sich teilenden Zelle ein zeitlicher Abstand der Aktivitätspeaks von CDK2- und MAP-Kinase von etwa 24 Stunden.

Dieser in der einzelnen transformierten und infizierten Zelle über eine längere Zeit im Zellzyklus hinweg beobachtete zeitgleiche Ablauf von Signaltransduktionswegen, die sich in der gesunden Zelle ausschließen, wurde von den Erfindern zum ersten mal erkannt und therapeutisch ausgenutzt. Dabei haben die Erfinder verstanden, dass die Selektivität des Substrates für transformierte und infizierte Zellen besonders gut gewährleistet ist, wenn die erfindungsgemäße Substanz derart ausgestaltet ist, dass diese einerseits sowohl mit einem beliebigen Enzym der Ras/Raf-, als auch andererseits mit einem beliebigen Enzym der CDK2-Signaltransduktionskaskade derart reagieren kann, dass dadurch eine biologische oder/und detektierbare Eigenschaft induziert wird.

Die Substanz kann jedoch auch so ausgestaltet werden, dass die in Rede stehende Eigenschaft dann induziert wird, wenn eine Reaktion mit Enzymen aus zumindest zwei anderen solchen Signaltransduktionskaskaden erfolgt, die in gesunden Zellen nicht zeitgleich ablaufen. Beispiele weiterer solcher Enzyme, finden sich unter: http://www.infobiogen.fr/services/ chromcancer/ oder http://www.ncbi.nlm.nih.gov/entrez/query. fcgi?CMD=search&DB=omim (Eingabe: "cancer"), wobei der Inhalt dieser Seiten durch Bezugnahme in die Anmeldung einbezogen ist.

Weitere Beispiele sind in dem Artikel von Weinberg und Hahn (a.a.O.) genannt.

Nach einer bevorzugten Weiterbildung stellt die erfindungsgemäße Substanz ein Substrat für die zumindest zwei Moleküle dar.

Dies hat den Vorteil, dass damit eine Substanz bereitgestellt wird, die direkt mit den zwei Molekülen in gewünschter Art und Weise reagiert, ohne dass zwischengeschaltete Faktoren berücksichtigt werden müssen. So kann die Substanz vorzugsweise zwei verschiedene Phosphorylierungsstellen aufweisen, wobei die eine bspw. spezifisch von der MAP-Kinase und die andere bspw. spezifisch von der CDK2-Kinase erkannt und phosphoryliert wird. Nur die im Wesentlichen zeitgleiche Reaktion des Substrates, d.h. die im Wesentlichen gleichzeitige Phosphorylierung sowohl der CDK2- als auch der MAP-Kinase-Phosphorylierungsstelle führt dann zur Induktion der biologischen oder/und detektierbaren Eigenschaft.

Die Substanz kann jedoch auch auf andere Art und Weise derart ausgestaltet sein, dass sie bei einer im Wesentlichen gleichzeitigen Reaktion mit den zwei Molekülen als Substrat direkt in eine aktive Form überführt wird, bspw. durch Freigabe von aktiven Zentren oder reaktiven Gruppen, die vor der Reaktion mit den zwei Molekülen sterisch unzugänglich oder chemisch inaktiv vorliegen.

Diese Ausgestaltung der erfindungsgemäßen Substanz als Substrat für die zwei Moleküle ist für einen Fachmann ohne größere Mühen zu bewerkstelligen. Bspw. sind die sich voneinander unterscheidenden CDK2- und MAP-Kinase-Phosphorylierungsstellen im Stand der Technik hinreichend bekannt: Die CDK2-Phosphorylierungsstelle ist bspw. in den Publikationen von Brown et al. (1999), The Structural Basis for Specificity of Substrate and Recruitment Peptides for Cyclin-dependent Kinases, Nat. Cell Biol., Vol. 1(7), Seiten 438 bis 443, und von Songyang et al. (1994), Use of an Oriented Peptide Library to Determine the Optimal Substrates of Protein Kinases, Curr. Biol., Vol. 4(11), Seiten 973 bis 982, beschrieben. Die Phosphorylierungsstelle für die MAP-Kinase ist bspw. in der Publikation von Songyang et al. (1996), A Structural Basis for Substrate Specificities of Protein Ser/Thr Kinases: Primary Sequence Preference of Casein Kinases I and II, NIMA, Phosphorylase Kinase, Calmodulindependent Kinase II, CDK5, and Erk1, Mol. Cell. Biol., Vol. 16(11), Seiten 6486 bis 6493, beschrieben. Die Phosphorylierungsstellen sind mittels üblicher Peptidsynthesen herstellbar.

Nach einer bevorzugten Weiterbildung ist die erfindungsgemäße Substanz derart ausgestaltet, dass bei einer Reaktion mit zumindest einem zellulären Faktor die Induktion der biologischen oder/und detektierbaren Eigenschaft modifiziert wird.

Unter zellulärem Faktor wird erfindungsgemäß jedes intrazelluläre Molekül, wie bspw. ein Protein mit einer definierten Aktivität, verstanden, das mit der Substanz indirekt oder direkt wechselwirkt und dadurch die Induktion der biologischen oder/und detektierbaren Eigenschaft modifiziert. Eine Modifizierung kann bedeuten, dass die Induktion der Eigenschaft durch die Wechselwirkung mit dem zellulären Faktor verstärkt wird, oder überhaupt dann erst stattfindet. Unter Modifizierung wird auch verstanden, dass die Induktion der Eigenschaft durch die Reaktion mit dem zellulären Faktor abgeschwächt wird, oder gänzlich ausbleibt.

Diese Maßnahme hat den Vorteil, dass die Induktion der biologischen oder/und detektierbaren Eigenschaft noch besser reguliert wird. Ferner kann die Substanz so ausgestaltet werden, dass diese nur mit einem solchen zellulären Faktoren reagiert, die in einer transformierten oder infizierten Zelle vorhanden sind und durch die Reaktion die Induktion der Eigenschaft verstärkt wird. Umgekehrt ist es auch möglich, dass die Substanz so ausgestaltet wird, dass eine Reaktion nur mit solchen Faktoren erfolgt, die in einer gesunden Zelle vorhanden sind. Durch die letztere Maßnahme wird die erfindungsgemäße Substanz in einer gesunden Zelle bspw. so verändert, dass eine wie auch immer gestaltete Induktion der biologischen oder/und detektierbaren Eigenschaft, die sich aufgrund unvorhersehbarer Ereignisse auch ohne das Auftreten der zwei Moleküle vollziehen könnte, verhindert wird. Die therapeutische bzw. diagnostische Eignung wird durch diese Sicherheitsmaßnahme weiter erhöht.

Vorzugsweise handelt es sich bei dem zellulären Faktor um proapoptotische oder antiapoptotische Moleküle oder um das Telomerase-Enzym.

Durch diese Maßnahme wird auf zweckmäßige Art und Weise eine Verbesserung der Selektivität und Spezifität der erfindungsgemäßen Substanz für transformierte oder/und infizierte Zellen gewährleistet. So ist bekannt, dass die Telomerase in transformierten Zellen besonders aktiv vorliegt, wohingegen in gesunden Zellen keine oder nur lediglich schwache Telomerase-Aktivität festgestellt werden kann. Vergleichbare Verhältnisse gelten für antiapoptotische Moleküle. Diese sind im Wesentlichen in transformierten Zellen, nicht jedoch bzw. in weit geringerem Maße in gesunden Zellen aktiv. Durch entsprechende Gestaltung der erfindungsgemäßen Substanz wird diese durch die Interaktion mit der Telomerase oder mit antiapoptotischen Molekülen in ihrer Fähigkeit zur Induktion einer biologischen oder/und detektierbaren Eigenschaft verstärkt.

Proapoptotische Mechanismen werden in transformierten oder in einer Vielzahl von infizierten Zellen frühzeitig inaktiviert. Dies erfolgt u.a. durch die Inaktivierung oder den Abbau von proapoptotischen Molekülen, die deshalb vorrangig nur in gesunden Zellen vorhanden bzw. aktiv sind. Durch geeignete Ausgestaltung der erfindungsgemäßen Substanz wird diese bei einer Reaktion mit proapoptotischen Molekülen derart verändert, dass die Induktion der biologischen oder/und detektierbaren Eigenschaft nicht möglich ist.

Die erfindungsgemäße Substanz ist vorzugsweise derart ausgestaltet, dass ein Eindringen oder eine Aufnahme in die Zelle oder/und Zellkompartimente ermöglicht ist.

Durch diese Maßnahme wird sichergestellt, dass die Substanz auch tatsächlich im Zellinneren bzw. in den gewünschten Zellkompartimenten, wie dem Cytoplasma oder dem Zellkern, die biologische oder/und detektierbare Eigenschaft induziert und nicht etwa aufgrund fehlender Aufnahme gar keine intrazelluläre Wirkung entfalten kann. Diese Ausgestaltung kann durch das Vorsehen eines membrangängigen Molekülabschnittes oder eines sonstigen Abschnittes erfolgen, der den passiven oder aktiven Transport der Substanz in die Zelle ermöglicht.

Dies kann auch durch das Vorsehen eines Bereiches oder Molekülabschnittes erfolgen, der die Affinität und das Internalisieren der Substanz an bzw. in die Zelle ermöglicht, wie bspw. durch einen an die Substanz gebundenen ggf. modifizierten Antikörper, ein Aptamer oder einen sonstigen Liganden. Ferner kann dadurch eine nochmals erhöhte Selektivität der Substanz geschaffen werden. So können bspw. Liganden vorgesehen werden, die das Eindringen oder die Aufnahme nur in ganz bestimmte transformierte oder infizierte Zellen ermöglichen, wie Zellen eines bestimmten Tumors oder solche, die von einem bestimmten Krankheitserreger infiziert wurden. Dazu können zelltypspezifische Oberflächenmarker, z.B. Tumorantigene ausgenutzt werden, an die die am erfindungsgemäßen Substrat vorgesehenen Liganden selektiv binden. Auch kann gemäß dieser bevorzugten Variante die Ausgestaltung der Substanz durch ein Verpacken in ein Transportvesikel erfolgen.

Nach einer bevorzugten Weiterbildung ist die erfindungsgemäße Substanz als niedermolekularer Wirkstoff ausgestaltet.

Im angelsächsischen Sprachgebrauch werden niedermolekulare Wirkstoffe auch als sogenannte "small molecules" bezeichnet. Es handelt sich hierbei um einen Sammelbegriff für chemische Substanzen mit Aktivitäten in biologischen Systemen, wobei deren Molekulargewichte i.d.R. unter etwa 1000 bis 1200 Dalton liegt, die vereinzelt aber auch größer sein können. Der Vorteil an dieser Maßnahme liegt darin, dass so die Substanz mittels etablierter Synthesemethoden in großmaßstablicher Art und Weise hergestellt werden kann und hinreichend stabil ist. Ferner können hier im Stand der Technik vorhandene chemisch definierte oder biologische Leitstrukturen, wie Peptide, verwendet werden und die Eigenschaften mit den Methoden des sogen. "rational drug designs" oder der sogen. "molecular evolution" oder "specificity evolution" durch chemische Synthese optimiert werden. Die Herstellung von niedermolekularen Wirkstoffen ist dem Fachmann geläufig; siehe Böhm et al. (2002), Wirkstoffdesign, Spektrum Akademischer Verlag, Heidelberg. Neueste Entwicklungen auf dem Gebiet der Herstellung niedermolekularer Wirkstoffe werden auch über das Internet zur Verfügung gestellt: http://www.nature.com/horizon/chemicalspace/highlights.html. Der Inhalt dieser Publikationen ist durch Bezugnahme Bestandteil der vorliegenden Anmeldung.

Die erfindungsgemäße Substanz ist vorzugsweise ein Peptid.

Diese Maßnahme ist deshalb von Vorteil, da so die erfindungsgemäße Substanz mittels bekannter Verfahren der Peptidchemie auf einfache Art und Weise hergestellt werden kann. So lässt sich die Substanz bspw. relativ einfach als Substrat für die zwei Moleküle ausgestalten, z.B. durch die Bereitstellung eines Abschnittes mit einer Phosphorylierungsstelle für die CDK2-Kinase und eines Abschnittes mit der Phosphorylierungsstelle für die MAP-Kinase. Ferner lassen sich somit im Stand der Technik bekannte membrangängige Peptidabschnitte leicht herstellen, wie vorzugsweise eine Sequenz aus Argininresten.

Ein weiterer Vorteil bei der Ausgestaltung der erfindungsgemäßen Substanz als Peptid liegt darin, dass sich dieses als Template bzw. als Leitstruktur für die weitere Entwicklung eines "small molecules" besonders eignet. So kann bspw. ein zu den zwei Molekülen affines Peptid mit den gewünschten biologischen Eigenschaften synthetisiert werden, über Standardverfahren ein Co-Kristall aus dem Peptid und den beiden Molekülen gewonnen werden und hieraus mit der Methode der molecular evolution" bzw. "specificity evolution" *in silico* eine entsprechende niedermolekulare Substanz abgeleitet und dann chemisch synthetisiert werden. Der vorstehend skizzierte Weg, in dem das Peptid als "Template" für ein entsprechendes "small molecule" und damit als eine Art Zwischenprodukt zur erfindungsgemäßen Substanz verwendet wird, ist dann für den Fachmann klar vorgezeigt.

Eine einfache Handhabung der Substanz wird dann auch bei dieser erfindungsgemäßen Weiterbildung über das Anbringen von solchen Abschnitten leicht ermöglicht, die eine Bindung an eine Affinitätssäule ermöglichen, wie bspw. einen aus Histidinresten bestehenden Abschnitt oder ein Tag bestehend aus der Glutathion-S-Transferase (GST). Diese Abschnitte bzw. Tags lassen sich bei einem Peptid besonders einfach vorsehen. Die einzelnen funktionellen Abschnitte können dann z.B. über verbindende Sequenzen, sogenannte "Linker" miteinander verbunden werden. Durch die Ausgestaltung als Peptid wird deshalb eine jeweils nach den gewünschten Eigenschaften flexible und einfache Herstellung der Substanz möglich.

Die vorstehend erwähnte einfache Herstellung von solchen Peptiden, mit denen über eine Reaktion mit zelleigenen Molekülen eine gewünschte Reaktion in der Zelle induziert werden kann, ist im Stand der Technik hinreichend beschrieben. So beschreiben bspw. Nguyen et al. (2004), Caged Phosphopeptides Reveal a Temporal Role for 14-3-3 in G1 Arrest and S-phase checkpoint Function, Nature Biotechnology, Vol. 22, Seiten 993 bis 1000, die Herstellung eines Peptidkonstruktes, das in biologische Zellen einbringbar ist und nach Aktivierung über UV-Bestrahlung eine Reaktion mit zellzyklusregulierenden Molekülen zeigt. Die dort präsentierten Daten belegen zusätzlich die Ausführbarkeit der vorliegenden Erfindung.

Ein weiterer Vorteil der Ausgestaltung der Substanz als Peptid liegt darin, dass diese dann mittels molekularbiologischer Methoden bspw. unter Verwendung eines Expressionsvektors in prokaryotischen oder eukaryotischen Expressionssystemen leicht hergestellt, mutiert oder auf sonstige Art und Weise verändert werden kann. Ein weiterer Gegenstand der Erfindung ist deshalb auch ein Expressionsvektor, der für ein entsprechendes erfindungsgemäßes Peptid kodiert. Selbstverständlich kann der erfindungsgemäße Expressionsvektor Abschnitte aufweisen, die eine Expression, u.U. auch zelltypspezifisch, ermöglichen oder fördern, wie Promotoren, Enhancer etc., oder auch Abschnitte, die eine Handhabung des Vektors im Labor ermöglichen, wie bspw. antibiotikaresistenzcodierende Abschnitte, Restriktionsschnittstellen, Polylinker etc.

Es ist auch denkbar, dass der erfindungsgemäße Expressionsvektor direkt als therapeutische oder/und diagnostische Substanz verwendet wird und durch geeignete Ausgestaltung in biologische Zellen einbringbar ist und dort exprimiert wird. Diese Ausgestaltung hat auch den Vorteil, dass Nukleinsäuren wesentlich stabiler und robuster als Proteine sind und nahezu unbegrenzt gelagert werden können. Die Herstellung des erfindungsgemäßen Expressionsvektors erfolgt mittels im Stand der Technik beschriebener Methoden. Als Beispiel für entsprechende Anleitungen wird die Abhandlung von Joseph Sambrook und David W. Russel (2001), Molecular Cloning - A Laboratory Handbook, Cold Spring Harbor Laboratory Press, Second Edition genannt, deren Inhalt durch Bezugnahme Bestandteil der vorliegenden Anmeldung ist.

Die Substanz ist vorzugsweise derart ausgestaltet, dass die biologische Eigenschaft unmittelbar oder mittelbar toxisch für die transformierte oder/und infizierte Zelle ist.

Dadurch wird eine selektiv nur für transformierte oder infizierte Zellen toxische Substanz geschaffen, die für gesunde Zellen weitgehend unschädlich ist, da nur die im Wesentlichen zeitgleiche Reaktion mit den zwei Molekülen zur Induktion der Toxizität führt, wohingegen eine Reaktion mit nur einem oder keinem der zwei Moleküle keine toxische Aktivität entfaltet.

Unter toxischer Eigenschaft wird eine solche Aktivität verstanden, die direkt oder indirekt zum Absterben der transformierten oder infizierten Zelle führt, bspw. durch Induktion der Apoptose, Nekrose oder Onkose, durch Hemmung des Stoffwechsels, der Signaltransduktion, des Proteasoms oder Transkriptionsaktivität, durch Interaktion mit dem Spindelapparat der Zelle etc. Unter toxischer Eigenschaft ist aber auch eine Aktivität zu verstehen, die zur Arretierung des Zellzyklus führt oder zur gezielten Aktivierung des Immunsystems oder Expression einer antigenen Determinante mit der Folge eines Angriffs auf die transformierte oder infizierte Zelle.

Die Phosphorylierungsstellen für die zwei Moleküle werden vorzugsweise in der Sequenz des p53-Moleküls oder Abschnitten hiervon bereitgestellt.

Unter dieser Maßnahme ist zu verstehen, dass die Sequenz oder eine oder mehrere Teilsequenzen des p53-Moleküls, vorzugsweise der humanen Variante, Bestandteil der erfindungsgemäßen Substanz ist und Phosphorylierungsstellen für die zwei Moleküle, bzw. die zwei Kinasen, aufweist, die in transformierten bzw. infizierten Zellen im Wesentlichen zeitgleich auftreten. Die Phosphorylierungsstellen können deshalb z.B. in die Sequenz des p53-Moleküls eingebettet sein oder die natürlicherweise im p53-Molekül vorhandenen Phosphorylierungsstellen darstellen oder ersetzen.

Das p53-Molekül ist ein Tumorsuppressorprotein, das in seiner Aktivität über Phosphorylierungsereignisse reguliert wird. So führt die Phosphorylierung des p53-Proteins zu einer Erhöhung von dessen Stabilität. Das p53-Protein wirkt dann als aktiver Transkriptionsfaktor und führt zu einer Aktivierung von zellzyklusarretierenden Proteinen, wie p21^{Cip1}, oder zur Einleitung der Apoptose.

Durch die Bereitstellung der Phosphorylierungsstellen für die beiden Kinasen in dem p53-Molekül oder funktionellen Abschnitten hiervon, z.B. von Phosphorylierungsstellen für die MAP- und CDK2-Kinase, wird deshalb ein Tool geschaffen, das p53-spezifische Aktivitäten entfalten kann. Dabei wird die Substanz so gestaltet, dass sich nur bei einer Phosphorylierung beider Phosphorylierungsstellen oder einer im Wesentlichen gleichzeitigen Phosphorylierung beider Phosphorylierungsstellen p53-spezifische Aktivitäten entfalten und es deshalb nur in transformierten oder infizierten Zellen zur Arretierung des Zellzyklus oder/und zur Apoptose kommt.

Nach einer erfindungsgemäßen Weiterbildung ist die Substanz derart ausgestaltet, dass die detektierbare Eigenschaft mittels bildgebender Verfahren detektierbar ist.

Eine solche Maßnahme kann bspw. durch die Ausgestaltung der Substanz als photoaktivierbares Molekül realisiert werden, das bei einer Reaktion mit den zwei Molekülen ein detektierbares Signal emittiert. Die Reaktion der erfindungsgemäßen Substanz mit den zwei Molekülen kann aber auch erst zur Aktivierung eines weiteren Moleküls führen, das dann eine detektierbaren Eigenschaft emittiert.

Bei der detektierbaren Eigenschaft kann es sich um emittierte Lumineszenz bzw. Fluoreszenz, Phosphoreszenz, Biolumineszenz, Radioaktivität, oder jedes andere detektierbare Signal handeln. Es ist ferner bspw. möglich, das Reaktionsprodukt, das aufgrund der Reaktion der erfindungsgemäßen Substanz mit den zwei Molekülen entstanden ist, durch die Verwendung von Antikörpern oder sonstigen Liganden direkt oder indirekt nachzuweisen.

Im Rahmen der bildgebenden Verfahren können Methoden wie Tomographie, FACS (fluoreszenzaktiviertes Zellsortieren), FRET (Fluoreszenz-Resonanz-Energietransfer), Fluoreszenzmikroskopie, Immunoblot, ELISA, radiologische Methoden etc. Verwendung finden.

Gegenstand der vorliegenden Erfindung ist auch eine Zusammensetzung, vorzugsweise eine pharmazeutische Zusammensetzung, die ausschließlich in einer transformierten oder/und infizierten Zelle eine biologische oder/und detektierbare Eigenschaft induziert.

Im Sinne der Erfindung bedeutet eine ausschließlich in transformierten oder/und infizierten Zellen induzierte Eigenschaft, dass eine Induktion in gesunden Zellen zumindest weitgehend wenn nicht vollständig vermieden wird bzw. sich eine Induktion in gesunden Zellen im Hinblick auf den therapeutischen bzw. diagnostischen Nutzen der Zusammensetzung tolerieren lässt.

Die Erfinder belegen anhand der ihnen vorliegenden Daten erstmals, dass ein gezielter und selektiver Angriff auf transformierte oder infizierte biologische Zellen möglich ist, wobei gesunde Zellen weitgehend unberührt bleiben. Ferner stellen die Erfinder erstmals eine Substanz bzw. eine Zusammensetzung bereit, die hochselektiv ausschließlich in transformierten oder/und infizierten Zellen eine biologische oder/und diagnostische Eigenschaft induzieren. Dies ist im Stand der Technik bislang nicht erreicht worden.

Die Zusammensetzung weist vorzugsweise die erfindungsgemäße Substanz bzw. den erfindungsgemäßen Expressionsvektor und ggf. einen pharmazeutisch akzeptablen Träger auf. Die Herstellung einer solchen pharmazeutischen Zusammensetzung ist im Stand der Technik hinreichend beschrieben. In diesem Zusammenhang wird auf die Publikation von Arthur A. Kibbe (2000), Handbook of Pharmazeutical Excipients, American Pharmaceutical Association and Pharmaceutical Press, Third Edition, verwiesen, deren Inhalt durch Bezugnahme Bestandteil der vorliegenden Anmeldung ist. Auch ist die Wahl der geeigneten Konzentration der Substanz in der Zusammensetzung ins Belieben des Fachmanns gestellt und lässt sich mittels einfacher Versuche, z.B. Titrationsversuche, leicht ermitteln. Auch muss die optimale Wirkstoffkonzentration in vielen Fällen individuell ermittelt werden und ist abhängig vom zu behandelnden Patienten.

Die erfindungsgemäße Zusammensetzung weist vorzugsweise Wirkungsverstärker auf. Darunter sind sämtliche Verbindungen zu verstehen, die die Induktion der biologischen oder/und detektierbaren Eigenschaft durch die erfindungsgemäße Substanz verstärken. Geeignete Wirkungsverstärker für eine Anwendung *in vitro* sind Tumorpromotoren, wie Phorbol-12-Myristat-13-Acetat (PMA), Ionomycin, für eine Anwendung *in vivo* sind dies Cytostatika, wie Herceptin oder Rituximab, oder Wachstumsfaktoren, wie G-CSF oder FGF. Die Wirkungsverstärker werden jeweils in geeigneten Konzentrationen eingesetzt, so dass das Auftreten der zumindest zwei Moleküle bzw. deren Aktivität ausschließlich in den transformierten und infizierten Zellen verstärkt wird, gesunde Zellen jedoch unbeeinflusst bleiben. Weitere geeignete Wirkungsverstärker in der erfindungsgemäßen pharmazeutischen Substanz sind bei vorgesehener therapeutischer Verwendung allgemein im Stand der Technik bekannte Cytostatika oder sonstige Wirkstoffe, die in der Therapie von Krebserkrankungen oder Infektionen verwendet werden. Auch sogenannte "Sensibilisierer", wie bispezifische Antikörper kommen als Wirkungsverstärker in Frage.

Vor diesem Hintergrund ist Gegenstand der vorliegenden Erfindung auch die Verwendung der erfindungsgemäßen Substanz bzw. des erfindungsgemäßen Expressionsvektors zur Herstellung einer pharmazeutischen Zusammensetzung zur Behandlung von transformierten und/oder infizierten biologischen Zellen.

Ein weiterer Gegenstand der Erfindung betrifft ein Verfahren zur Diagnose einer Tumorerkrankung oder/und Infektion bei einem Lebewesen, das folgende Schritte aufweist: (a) Bereitstellung einer zu untersuchenden biologischen Probe; (b) Untersuchung des Auftretens von Molekülen in einzelnen Zellen der biologischen Probe, und (c) Korrelation des Auffindens von im Wesentlichen gleichzeitig auftretenden Molekülen in einzelnen Zellen der Probe, die ausschließlich in einer transformierten und/oder infizierten biologischen Zelle im Wesentlichen gleichzeitig auftreten, mit einer positiven Diagnose.

Erfindungsgemäß umfasst eine biologische Probe sowohl isolierte Zellen und Gewebe als auch ganze Organismen, d.h. auch ein menschliches und tierisches Lebewesen. Das diagnostische Verfahren kann also an isoliertem biologischem Material im Labor, also *in vitro,* aber auch im oder am lebenden Organismus, d.h. *in vivo* oder *in situ,* durchgeführt werden.

Ein entscheidender Vorteil dieses Verfahrens liegt darin, dass nach einer positiven Diagnose auch Informationen darüber erhalten werden, welche Moleküle in den transformierten oder infizierten Zellen gegenüber gesunden Zellen gleichzeitig vorhanden sind. Dies ermöglicht dem behandelnden Arzt anschließend einen gezielten Einsatz von Therapeutika, wie sogar herkömmlichen Cytostatika, die spezifisch an den zwei Molekülen angreifen bzw. in die entsprechenden Signaltransduktionswege eingreifen.

Die Untersuchung in Schritt (b) erfolgt vorzugsweise mittels des sogenannten Single Cell Profilings.

Diese Methode, die bspw. in Irish et al., a.a.O. beschrieben ist, erlaubt eine einzelzellspezifische Untersuchung von intrazellulären Ereignissen, wie das Erfassen von in der einzelnen Zelle gleichzeitig auftretenden Molekülen und deren Aktivitäten. So lässt sich bspw. in einer einzelnen Zelle die Aktivität von Enzymen bzw. Kinasen messen. Die Erzeugung von Artefakten, die durch die Untersuchung an Zellkulturen oder Zellverbänden aufgrund der hier angewendeten Methoden induziert werden können, bspw. durch das Synchronisieren der Zellen im Zellzyklus, werden durch das Single Cell Profiling vermieden. Die zu untersuchenden Zellen können vielmehr vor dem Hintergrund des natürlichen, physiologischen Zellzyklus auf Einzelzellebene analysiert werden.

In Schritt (a) erfolgt vorzugsweise eine Stimulierung der biologischen Probe mittels eines Tumorpromotors, vorzugsweise mittels Phorbol-12-Myristat-13-Acetat (PMA), Ionomycin, eines Cytostatikums, vorzugsweise Herceptin oder Rituximab, oder eines Wachstumsfaktors, wie vorzugsweise G-CSF oder FGF.

Die Erfinder haben erkannt, dass nach einer Stimulierung der Zellen *in vitro* mit einem solchen Tumorpromotor, oder *in vivo* mit Wachstumsfaktoren oder Cytostatika eine Unterscheidung von transformierten und/oder infizierten biologischen Zellen gegenüber gesunden Zellen besonders gut möglich ist. Die Substanzen Herceptin und Rituximab bspw. aktivieren den Ras/Raf-Weg über Her2/neu bzw. CD20 und verstärken damit die Kinaseaktivität in transformierten und infizierten Zellen nochmals. So wurde von den Erfindern nach einer entsprechenden Stimulation eine deutliche zeitlich unterschiedliche Induktion von Kinaseaktivtäten, bspw. der MAK- und CDK2-Kinaseaktivitäten, in gesunden CD34-Zellen gegenüber AML-Tumorzellen auf Einzelzellebene beobachtet, über die dann die Diagnose einer Tumorerkrankung oder einer Infektion erfolgen kann.

In dem vorstehenden Vorfahren erfolgt in Schritt (c) vorzugsweise eine Inkubation der biologischen Probe mit der oben beschriebenen erfindungsgemäßen Substanz oder/und dem oben beschriebenen erfindungsgemäßen Expressionsvektor, wobei dann in Schritt (b) die detektierbare Eigenschaft detektiert wird.

Ein weiterer Gegenstand der vorliegenden Erfindung betrifft ein Verfahren zur Behandlung einer Tumorerkrankung oder/und Infektion bei einem Lebewesen, wobei diesem die oben erläuterte erfindungsgemäße Substanz oder/und der oben erläuterte erfindungsgemäße Expressionsvektor appliziert wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

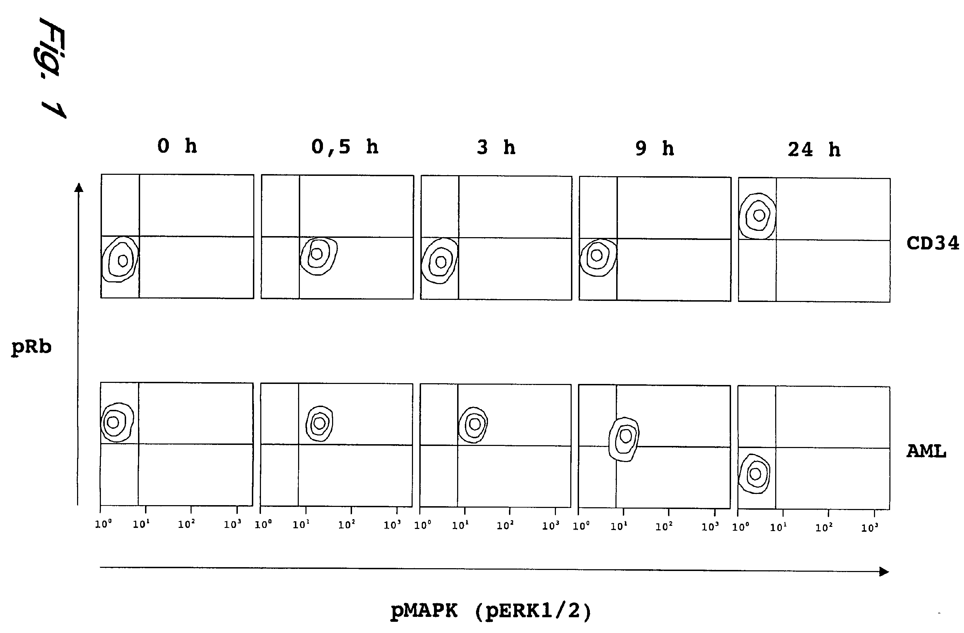

Die Gegenstände der vorliegenden Erfindung werden nun anhand von Ausführungsbeispielen erläutert, die rein illustratorischen Charakter haben und zu keinerlei Einschränkung der erfindungsgemäßen Lehre führen. Darin wird auf die beiliegende Figur 1 Bezug genommen:
- Fig. 1:: Gleichzeitiger Ablauf der Ras/Raf- und der CDK2-Signalwege in transformierten Zellen

### Ausführunqsbeispiele

### Beispiel 1: Differentielle Signaltransduktion in gesunden Zellen und transformierten Zellen

CD34-positive Blut-Stammzellen wurden durch "Magnetic Cell Sorting" (MACS) isoliert. Aus dem peripheren Blut eines Patienten mit akuter myeloischer Leukämie (AML) M5 bei > 80 % Blasten wurden AML-Tumorzellen ohne weitere Manipulation gewonnen.

Die Zellen wurden mit PMA/Ionomycin aktiviert und damit in den Zellzyklus entlassen. Zu verschiedenen Zeiten wurden die Zellen in Formaldehyd fixiert und die Membranen mit Methanol permeabilisiert.

Anschließend wurde in beiden Zelltypen die Aktivität der Faktoren des Ras/Raf-Signalweges anhand der Phosphorylierung der MAP-Kinase (pMAPK bzw. pERK1/2), und die Aktivität der Faktoren des CDK2-Signalweges anhand der Phosphorylierung des Retinoblastom-Proteins (pRB), einem Substrat der CDK2-Kinase, untersucht.

Hierzu wurde die Methode der Durchflusscytometrie verwendet, wobei einzelne Zellen vermessen wurden. Die Methode ist ausführlich beschrieben in Irish et al. (a.a.O.). Die permeabilisierten Zellen wurden dafür mit Anti-Phospho-Rb/PE-Anti-Maus inkubiert, einem Antikörper, der spezifisch an phosphoryliertes Rb-Protein bindet, gefolgt von einer Inkubation mit FITCkonjugiertem Anti-MAP-Kinase-Antikörper, einem Antikörper, der spezifisch phosphorylierte MAP-Kinase (ERK 1/2) erkennt.

Das Ergebnis dieses Experimentes ist schematisch in Fig. 1 gezeigt. Hier sind repräsentative zweidimensionale Plots aus der Durchflusscytometrie skizziert. Auf der X-Achse ist die zunehmende Phosphorylierung der MAP-Kinase und auf der Y-Achse die zunehmende Phosphorylierung von Rb dargestellt. In den Plots ist ein Messkreuz eingezeichnet, das eine Orientierung der Veränderung der ausgelesenen Signale vereinfacht.

Zu sehen ist, dass in gesunden CD34-Zellen nach 30 min, was einer frühen G1-Phase entsprechen könnte, die MAP-Kinase phosphoryliert vorliegt. Dies zeigt sich in einer Verschiebung des gemessenen Signals nach rechts. Rb hingegen wird nicht phosphoryliert, eine Verschiebung des gemessenen Signals nach oben erfolgt nicht.

In den gesunden CD34-Zellen kommt es erst nach ca. 24 Stunden, was in etwa der späten G1- bzw. frühen S-Phase entsprechen könnte, zur Phosphorylierung von Rb, wohingegen die MAP-Kinase schon wieder dephosphoryliert vorliegt (Fig. 1, obere Reihe). Zu noch späteren Messzeitpunkten, die in der Fig. 1 nicht dargestellt sind, findet man Rb wieder dephosphoryliert.

Diese Beobachtung in den gesunden Zellen steht im Einklang mit den Kenntnissen im Stand der Technik: In der frühen G1-Phase kommt es zur Aktivierung des Ras/Raf-Weges und damit zur Phosphorylierung und Aktivierung der MAP-Kinase. In der späten G1- bzw. frühen S-Phase ist die MAP-Kinase wieder inaktiv und damit dephosphoryliert. Hingegen kommt es zu diesem Zeitpunkt zu einer Aktivierung des CDK2-Signalweges und damit zur aktiven CDK2-Kinase, die zuerst als cdk2/Cyclin E- und dann als cdk2/Cyclin A-Komplex vorliegt und verschiedene Substrate, wie das pRB-Protein, phosphoryliert. Die MAP-Kinase und die CDK2-Kinase sind jedoch niemals gleichzeitig aktiv.

Völlig unerwartet und hier erstmals gezeigt, sind die in den transformierten Zellen ablaufenden Phänomene: Die Kinetik der Aktivierung der Raf/Raf- und CDK2-Signaltransduktionsketten ist im Vergleich zu jener in gesunden Zellen stark verändert. Das Rb-Protein liegt bereits zum ersten Messzeitpunkt (t = 0 h) phosphoryliert vor, woraus geschlussfolgert werden kann, das die CDK2-Kinase bereits in aktiver Form vorliegt. Dies ist durch eine Versetzung des Messsignals nach oben dargestellt, wobei sich die Phosphoform des Rb-Proteins bspw. radioimmunologisch nachweisen lässt. Bereits 30 min nach der Stimulation ist neben Rb gleichzeitig auch die MAP-Kinase phosphoryliert. Dies zeigt sich in einer Verschiebung des gemessenen Signals nach rechts. Diese gleichzeitige Phosphorylierung des Rb-Proteins und der MAP-Kinase lässt sich über lange Zeiten des Messzeitraums nachweisen. Erst nach 24 h liegen sowohl das Rb-Protein als auch die MAP-Kinase wieder dephosphoryliert vor (Fig. 1, untere Reihe).

Dieser Unterschied zwischen gesunden bzw. transformierten Zellen lässt sich auch ohne vorherige Stimulierung der Zellen beobachten, wobei dann die gleichzeitige Phosphorylierung des Rb-Proteins und der MAP-Kinase in den malignen Zellen etwas schwächer zu sehen ist.

In den transformierten Zellen findet man deshalb überraschenderweise bereits unmittelbar nach der Entlassung dieser Zellen in den Zellzyklus ein im Wesentlichen zeitgleiches Ablaufen sowohl des Ras/Raf-Signalweges, als auch des CDK2-Signalweges.

Sowohl aktive MAP-Kinase als auch aktive CDK2-Kinase lassen sich im Wesentlichen zeitgleich in den transformierten AML-Zellen nachweisen. Das in gesunden Zellen beobachtete, über den Zellzyklus hinweg zeitlich versetzte Erscheinen von aktiver MAP-Kinase (frühe Messung, 0,5 h) und aktiver CDK2-Kinase (späte Messung, 24 h) ist deshalb in transformierten Zellen aufgehoben. Beide Aktivitäten sind zeitgleich vorhanden.

### Beispiel 2: Herstellung der erfindungsgemäßen Substanz

### (a) als niedermolekularer Wirkstoff ("small molecule")

Die Herstellung von niedermolekularen Wirkstoffen ist grundsätzlich im Stand der Technik beschrieben und gehört zum Handwerkszeug eines klinischen Chemikers; vgl. Böhm et al. (a.a.O.). Insbesondere sind umfangreich Verfahren beschrieben, mit denen solche niedermolekularen Wirkstoffe hergestellt werden können, die mit Signaltransduktionsmolekülen, wie Kinase-Inhibitoren reagieren: Buchdunger et al. (1995), Selective Inhibition of the Platelet-Derived Growth Factor Signal Transduction Pathway by a Protein-Tyrosine Kinase Inhibitor of the 2-Phenylaminopyrimidine Class, Proc. Natl. Acad. Sci. USA, Vol. 92, Seiten 2558 bis 2562; Druker et al. (1996), Effects of a Selective Inhibitor of the Abl Tyrosine Kinase on the Growth of Bcr-Ab1 positive Cells, Nat. Med., Vol. 2, Seiten 561 bis 566; Schindler et al. (2000), Structural Mechanism for STI-571 Inhibition of Abelson Tyrosine Kinase, Science, Vol. 289, Seiten 1938 bis 1942. Eine weitere Publikation beschreibt die Herstellung eines "Small molecules" am Beispiel von Imatinib: Thomas Fischer (2002), Der Signalhemmer Imatinib Mesilat (STI571) - Wirkprinzip und klinische Anwendung, UNI-MED Verlag, Bremen.

Der Inhalt dieser Publikationen ist durch Bezugnahme Bestandteil der vorliegenden Anmeldung.

Durch die Anwendung der in den vorstehenden Publikationen beschriebenen Verfahren kann der Fachmann die erfindungsgemäße Substanz ohne unzumutbaren Aufwand herstellen. So lassen sich bspw. ausgehend von vorkonstruierten Peptiden als Leitstrukturen mit Methoden der molecular evolution" bzw. "specificity evolution" kleine Moleküle konstruieren, die Abschnitte aufweisen, über die ein selektives In-Kontakt-treten mit bestimmten zellulären Kinasen erfolgt. Diese Abschnitte, die von den Peptidleitstrukturen abgeleitet werden, wechselwirken bspw. mit der ATP-Bindestelle oder dem aktiven Zentrum der Kinasen. Dabei kann das Molekül so gestaltet werden, dass es nur bei einer im Wesentlichen zeitgleichen Wechselwirkung mit den ATP-Bindestellen oder aktiven Zentren der MAP-Kinase und der CDK2-Kinase zu einer Aktivierung kommt, die zur Induktion einer Toxizität oder eines detektierbaren Signals führt. Hierzu benötigt man u.U. die Kristallstrukturen der MAP-Kinase und der CDK2-Kinase, die in öffentlich zugänglichen Datenbanken abgelegt sind.

### (b) als Peptid

Die erfindungsgemäße Substanz kann mittels gängiger Peptidsyntheseverfahren hergestellt werden, dass sich folgende Struktur ergibt: Histidin-Reste - membranpermeable Sequenz - Linker - CDK2-Substrat - Linker - MAP-Kinase-Substrat. Der N-Terminus liegt auf der linken Seite, der C-Terminus auf der rechten Seite. Eine denkbare Aminosäuresequenz lautet: HHHHHH-RRRRRRRRR-GG-HHASPRK-GG-TGPLSPGPF. In dieser Darstellung ist der Standardeinbuchstabencode für Aminosäuren verwendet. Diese Sequenz kann so modifiziert werden, dass es bei einer im Wesentlichen gleichzeitigen Phosphorylierung der beiden Substrate zur Aktivierung der Substanz kommt, wodurch eine Toxizität oder ein detektierbares Signal induziert wird. Um dies zu gewährleisten, können weiter Abschnitte oder Moleküle bzw. Molekülabschnitte vorgesehen sein, die bei gleichzeitiger Phosphorylierung der Substanz aktiviert werden.

Die Funktionsfähigkeit der Substanz kann in einem Mausmodell überprüft werden. Dieses ist in der Publikation Traggiai et al. (2004), Development of a Human Adaptive Immune System in Cord Blood Cell-transplanted Mice, Science, Vol. 304(5667), Seiten 104 bis 107, beschrieben, durch das die doppelte Phosphorylierung der Substanz in transformierten Zellen nachgewiesen werden kann. Diese Publikation wird durch Bezugnahme Inhalt der vorliegenden Anmeldung.

In diesem Modell werden Mäuse mit gesundem humanem Immunsystem generiert. Durch eine Modifizierung dieses Modells lassen sich Mäuse mit humaner AML generieren, in denen die doppelte Phosphorylierung der erfindungsgemäßen Substanz gezeigt werden kann.

Denkbar sind selbstverständlich andere Ausgestaltungen. Bspw. werden die Substratabschnitte so ausgestaltet, dass es bei einer enzymatischen Umsetzung an den Substratabschnitten zur Induktion einer toxischen Aktivität kommt.

### Beispiel 3: Diagnose einer Tumorerkrankung mittels der erfindungsgemäßen Substanz

Einem auf Leukämie zu untersuchenden Patienten wird Blut entnommen und ggf. nach im Stand der Technik bekannten Verfahren behandelt bzw. kultiviert.

Daraufhin erfolgt eine Inkubation der Zellen mit der aus dem Beispiel 2 gewonnenen Substanz. Diese Substanz ist so gestaltet, dass sie bei gleichzeitigem Vorhandensein der MAP-Kinase und der CDK2-Kinase doppelt phosphoryliert wird. Beim Vorliegen nur einer der beiden Kinasen oder einem deutlich zeitlich versetztem Erscheinen der beiden Kinasen erfolgt lediglich eine einfache Phosphorylierung der Substanz.

Hierzu wird die Substanz als "Biosensor" zur Verwendung in der FRET- (Fluoreszenz-Resonanz-Energietransfer) Technologie ausgestaltet. Es werden geeignete FRET-Paare vorgesehen, bspw. Coumarin und Fluorescein, so dass es bei einer Doppelphosphorylierung der Substanz zu einer Konformationsänderung kommt, dadurch beide FRET-Paare in räumliche Nähe zueinander gebracht werden und ein detektierbares Signal emittiert wird. Die Konstruktion dieser Substanz gehört zum Fachwissen eines Spezialisten, wobei hierfür geeignete Verfahren bereits in Form von kommerziell erhältliche Konstruktionskits zur Verfügung stehen. Ein Beispiel hierfür ist der Z'-LYTE™-Assay der Firma Invitrogen, der unter http://invitrogen.com beschrieben ist. Der Inhalt der Beschreibung dieses Assays ist durch Bezugnahme Bestandteil der vorliegenden Anmeldung.

Die Zellen werden nach der Inkubation lysiert. Das Lysat wird mit Protease behandelt. Anschließend wird das FRET-Signal ausgelesen. Hier kann auch eine Anwendung im Durchflusscytometer und ein Single-Cell-Profiling (vgl. Irish et al., a.a.O.) durchgeführt werden.

Bei einer Detektion eines Signals als Hinweis auf eine Doppelphosphorylierung erfolgt eine positive Diagnose.

Ein weiteres geeignetes Verfahren zur Herstellung der erfindungsgemäßen diagnostischen Substanz ist beschrieben in Chi-Wang Lin und Alice Y. Ting (2004), A Genetically Encoded Fluorescent Reporter of Histone Phosphorylation in Living Cells, Angew. Chem. Int. Ed., Vol. 43, Seiten 2940 bis 2943. Der Inhalt der Publikation ist durch Bezugnahme Bestandteil der vorliegenden Anmeldung.

## Patentansprüche

1. Therapeutische oder/und diagnostische Substanz, die mittelbar oder unmittelbar mit zumindest zwei Molekülen, die ausschließlich in einer transformierten oder/und infizierten biologischen Zelle im Wesentlichen zeitgleich auftreten, derart reagiert, dass sie eine biologische oder/und detektierbare Eigenschaft induziert.

2. Substanz nach Anspruch 1, **dadurch gekennzeichnet, dass** die Moleküle zelluläre Enzyme, insbesondere Kinasen, die in die Regulation des Zellzyklus involviert sind, darstellen.

3. Substanz nach Anspruch 1 oder 2, **dadurch gekennzeichnet, dass** eines der zwei Moleküle ein Enzym der Ras/Raf-Signaltransduktionskette darstellt, und das andere der zwei Moleküle ein Enzym der CDK2-Signaltransduktionskette darstellt.

4. Substanz nach einem der vorherigen Ansprüche, **dadurch gekennzeichnet, dass** diese ein Substrat für die zumindest zwei Moleküle darstellt.

5. Substanz nach einem der vorherigen Ansprüche, **dadurch gekennzeichnet, dass** diese zumindest zwei verschiedene Phosphorylierungsstellen aufweist, wobei die eine die Phosphorylierungsstelle für das eine der zwei Moleküle darstellt, und die andere die Phosphorylierungsstelle für das andere der zwei Moleküle darstellt.

6. Substanz nach einem der vorherigen Ansprüche, **dadurch gekennzeichnet, dass** diese derart ausgestaltet ist, dass bei einer Reaktion mit zumindest einem zellulären Faktor die Induktion der biologischen oder/und detektierbaren Eigenschaft modifiziert wird.

7. Substanz nach Anspruch 6, **dadurch gekennzeichnet, dass** der zelluläre Faktor ausgewählt ist aus den proapoptotischen Molekülen, antiapoptotischen Molekülen und der Telomerase.

8. Substanz nach einem der vorherigen Ansprüche, **dadurch gekennzeichnet, dass** diese derart ausgestaltet ist, dass ein Eindringen oder eine Aufnahme in eine biologische Zelle oder/und Zellkompartimente ermöglicht ist.

9. Substanz nach einem der vorherigen Ansprüche, **dadurch gekennzeichnet, dass** diese einen niedermolekularen Wirkstoff ("small molecule") darstellt.

10. Substanz nach einem der Ansprüche 1 bis 8, **dadurch gekennzeichnet, dass** diese ein Peptid darstellt.

11. Substanz nach einem der Ansprüche 8 bis 10, **dadurch gekennzeichnet, dass** die Ausgestaltung über das Vorsehen eines membrangängigen Abschnittes, vorzugsweise einer Sequenz aus Argininresten, erfolgt.

12. Substanz nach einem der vorherigen Ansprüche, **dadurch gekennzeichnet, dass** diese derart ausgestaltet ist, dass eine Bindung an eine Affinitätssäule ermöglicht ist.

13. Substanz nach Anspruch 10, **dadurch gekennzeichnet, dass** die Ausgestaltung über das Vorsehen von Histidinresten oder/und eines GST-Tags erfolgt.

14. Substanz nach einem der vorherigen Ansprüche, **dadurch gekennzeichnet, dass** die biologische Eigenschaft unmittelbar oder mittelbar toxisch für eine transformierte oder/und infizierte biologische Zelle ist.

15. Substanz nach einem der Ansprüche 5 bis 14, **dadurch gekennzeichnet, dass** die Phosphorylierungsstellen in der Sequenz des p53-Proteins oder Abschnitten hiervon bereitgestellt werden.

16. Substanz nach einem der vorherigen Ansprüche, **dadurch gekennzeichnet, dass** diese derart ausgestaltet ist, dass die detektierbare Eigenschaft mittels bildgebender Verfahren detektierbar ist.

17. Expressionsvektor, der für ein Peptid nach einem der Ansprüchen 10 bis 16 kodiert.

18. Zusammensetzung, die ausschließlich in einer transformierten oder/und infizierten Zelle eine biologische oder/und detektierbare Eigenschaft induziert.

19. Zusammensetzung nach Anspruch 18, **dadurch gekennzeichnet, dass** diese die Substanz nach einem der Ansprüche 1 bis 16 oder/und den Expressionsvektor nach Anspruch 17 aufweist.

20. Zusammensetzung nach Anspruch 18 oder 19, **dadurch gekennzeichnet, dass** es sich um eine pharmazeutische Zusammensetzung handelt, die einen pharmazeutisch akzeptablen Träger aufweist.

21. Zusammensetzung nach einem der Ansprüche 18 bis 20, **dadurch gekennzeichnet, dass** diese zusätzlich Wirkungsverstärker, vorzugsweise Tumorpromotoren, wie Phorbol-12-Myristat-13-Acetat (PMA) oder Ionomycin, Cytostatika, wie Herceptin oder Rituximab, oder Wachstumsfaktoren, wie G-CSF oder FGF, aufweist.

22. Verwendung einer Substanz nach einem der Ansprüche 1 bis 16 oder/und des Expressionsvektors nach Anspruch 17 zur Herstellung einer pharmazeutischen Zusammensetzung zur Behandlung von transformierten oder/und infizierten biologischen Zellen.

23. Verfahren zur Diagnose einer Tumorerkrankung oder/und einer Infektion bei einem Lebewesen, das folgende Schritte aufweist:
(a) Bereitstellung einer zu untersuchenden biologischen Probe;
(b) Untersuchung des Auftretens von Molekülen in einzelnen Zellen der biologischen Probe, und
(c) Korrelation des Auffindens von im Wesentlichen gleichzeitig auftretenden Molekülen in einzelnen Zellen der Probe, die ausschließlich in einer transformierten und/oder infizierten biologischen Zelle im Wesentlichen gleichzeitig auftreten, mit einer positiven Diagnose.

24. Verfahren nach Anspruch 23, **dadurch gekennzeichnet, dass** die Untersuchung in Schritt (b) mittels Single Cell Profilings erfolgt.

25. Verfahren nach Anspruch 23 oder 24, **dadurch gekennzeichnet, dass** in Schritt (a) eine Stimulierung der biologischen Probe mittels eines Tumorpromotors, vorzugsweise Phorbol-12-Myristat-13-Acetat (PMA) oder Ionomycin, oder eines Cytostatikums, vorzugsweise Herceptin oder Rituximab, oder eines Wachstumsfaktors, vorzugsweise G-CSF oder FGF, erfolgt.

26. Verfahren nach einem der Ansprüche 23 bis 25, **dadurch gekennzeichnet, dass** in Schritt (a) eine Inkubation der biologischen Probe mit der Substanz nach einem der Ansprüche 1 bis 16 oder/und dem Expressionsvektor nach Anspruch 17 erfolgt, und in Schritt (b) die detektierbare Eigenschaft detektiert wird.

27. Verfahren zur Behandlung einer Tumorerkrankung oder/und einer Infektion bei einem Lebewesen, wobei diesem die Substanz nach einem der Ansprüche 1 bis 16 oder/und der Expressionsvektor nach Anspruch 17 oder/ und die Zusammensetzung nach einem der Ansprüche 18 bis 21, appliziert wird.
